# EUROPEAN PATENT SPECIFICATION

(11) **EP 1 874 413 B1**
(45) Date of publication and mention of the grant of the patent: **07.12.2016**
(21) Application number: 06733300.5
(22) Date of filing: 18.04.2006
(51) Int. Cl.: A63B 23/18, A61M 16/20

(54) **AN ADJUSTABLE RESPIRATORY PRESSURE DEVICE**
VERSTELLBARE ATEMDRUCKVORRICHTUNG
DISPOSITIF DE PRESSION RESPIRATOIRE REGLABLE

(30) Priority: 19.04.2005 SE 0500862
(43) Date of publication of application: 09.01.2008
(73) Proprietor: Eliasson, Kenneth, 97441 Luleå (SE)
(72) Inventor: Eliasson, Kenneth, 97441 Luleå (SE)
(74) Representative: Brann AB
(86) International application number: PCT/SE2006/000444
(87) International publication number: WO 2006/112773

(56) References cited:
- EP-A1- 0 372 148
- EP-A1- 0 372 148
- WO-A1-96/40376
- WO-A1-96/40376
- WO-A1-98/56468
- WO-A1-98/56468
- WO-A2-01/76671
- WO-A2-01/76671
- GB-A- 933 234
- US-A- 569 777
- US-A- 4 770 413
- US-A- 4 770 413

## Description

### TECHNICAL FIELD

The invention relates to respiratory pressure devices. Such devices are used in rehabilitation of patients suffering from breathing disorder or patients gone through an operation of the respiratory organs.

### BACKGROUND ART

There is a multitude of breathing disorders, such as lung injuries or infections in the respiratory system, which makes it suitable to use a respiratory device in order to rehabilitate a patient. The respiratory system comprises mouth, nose, trachea, lungs, and diaphragm. Another example when such rehabilitation is considered is after an operation, such as a lung operation, and/or after a long period at a hospital when a respirator was used to enable the patient to breath. It is common that such treatment is started at a hospital where medical trained personal instruct and assist the patient. The equipment commonly used at the hospital is advanced and relative expensive.

There are a number of known apparatus intended to enable a patient to continue rehabilitation at home.

EP 0,678,306 shows an example of such an apparatus. The apparatus is a single user respiratory therapy device including a pressure range monitoring unit which provides the patient with visual feed-back to monitor the correct use of the device for enhancing the benefits of positive expiratory pressure therapy. A remaining problem is that dismantling and assembling the apparatus is difficult, especially for the patient who lacks experience of the operation. The apparatus comprises two valves, which makes the manufacturing of the apparatus expensive and it also suggests that the patient should have access to spare valves at home. The apparatus comprises a scale positioned on the outside of the cover of the apparatus, which the patient use to adjust a breathing resistance, but the patient can not see the size of the adjusted opening between two opposite located holes in two opposite circle formed details which turns as the scale on the cover is adjusted. Another disadvantage is that the resulting opening is hidden from the patient in the inner of the apparatus.

WO 0,176,671 describes an apparatus for performing positive pressure therapy alone or in combination with an aerosol delivery apparatus. The positive pressure apparatus includes a positive pressure valve having a continuously variable respiratory window. The positive pressure valve may be associated with a patient respiratory system.

WO98/56468, WO96/40376 and EP0372148 are also part of the prior art.

A remaining problem with the existing apparatuses and devices is that they comprise a fairly large amount of components which makes cleaning of the apparatuses a complex operation.

### SUMMARY OF THE INVENTION

An object of the invention is to present a device for an adjustable the device is intended for respiratory therapy and is, easier to adjust regarding a positive expiratory pressure, easier to disassemble and clean, and less costly to produce compared to previous known devices.

This object is achieved by a device comprising a first and second part, which are at least partly cylindrically shaped, an inner surface of the first part which surrounds an outer surface of the second part. The surfaces are cylindrically shaped. The two parts may be revolved in respect to each other by a user and the two parts may be dismounted from each other. The surface of the first at least partly cylindrically shaped part comprises a mainly triangular hole. The surface of the second at least partly cylindrically part comprises a mainly triangular hole. The two holes are adapted to be positioned opposite each other during respiratory therapy where they form an opening of variable size. The opening is visible for the patient. The patient may feel a current length of an indicating bar with a finger that corresponds to the size of the opening.

As the main body of the device consists of only two parts, dismountable from each other, the device is easy to clean, this in contrast to known respiratory therapy apparatuses, which often comprises valves and/or membranes. It is straight forward to instruct a patient how to dismantle a device according to the invention, clean the device and assemble the device. The previous known therapy apparatuses are often of such complexity that tools are needed to dismantle them, this in contrast to the device which is dismantled by means of the patient's hands.

The device comprises a scale in order to adjust the size of the opening. The scale comprises a number of bars of increasing length.

The indications are positioned on even distances from each other. The positive expiratory pressure is adjusted by rotating the two parts in order to increase the pressure. Adjusting two neighboring bars compared to a reference line, or similar, results in a progressive increase of expiratory pressure, when the bar adjusted to is longer than the bar adjusted from. In an alternative embodiment the adjustment from a shorter bar to a longer bar corresponds to an increase of the area of the opening 12, which means a reduction of expiratory pressure. An advantage is that the indications, which are not depending on visible numerical references, facilitates the instruction of the patient in how to increase the pressure in a progressive manner during the time of therapy. Therapy is typically performed repeatedly during several days according to a scheme individually adapted to the patient.

Yet another advantage is that, in addition to visually reading indications, adjustment of the positive expiratory pressure may be performed as the patient interprets the scale by means of the fingers. This is a particular advantage for patients with defective vision.

### BRIEF DESCRIPTION OF THE DRAWINGS

The present invention will be described in more detail in connection with the enclosed drawings.
Figure 1 shows a device for respiratory therapy according to the invention. The device comprises two parts dismountable from each other.
Figure 2 shows a detail of the scale / indications comprising bars of increasing length.
Figure 3 shows a detail of the mainly triangular hole of the first part which surrounds the mainly triangular hole of the second part where the form an opening of variable size.

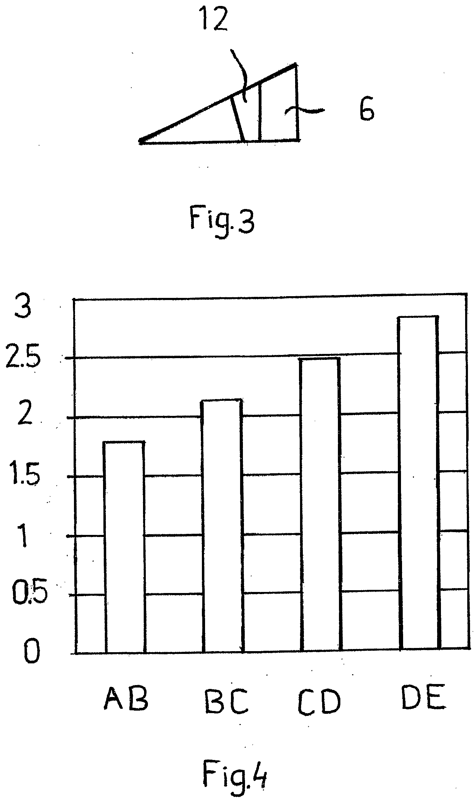

Figure 4 is a bar diagram. Each bar shows an increase/decrease of the area of the opening between neighboring indications of the scale. The figure shows that the enlargement of the opening increases in a progressive manner as the two parts are turned in the suggested direction. And opposite that the reduction of size increases as the two parts are turned in the opposite suggested direction. This is used when instructing the patient regarding how to proceed with the therapy. The patient is instructed to commence therapy with a large opening - for instance indicated by the largest bar - relating to a small positive expiratory pressure, and then gradually reduce the size of the opening during the period of therapy.

### DETAILED DESCRIPTION OF THE INVENTION

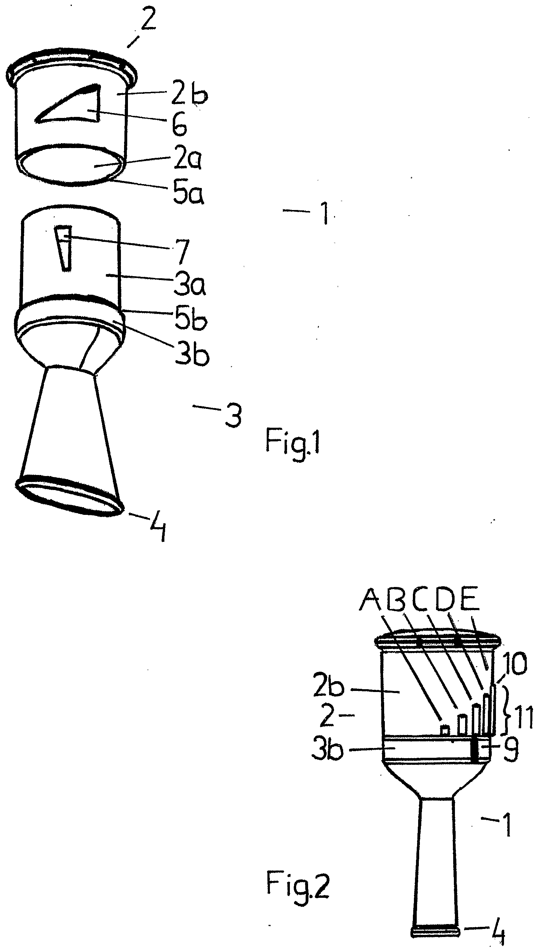

Figure 1 shows a device for respiratory therapy. The device comprises a first 2 and a second part 3, which are at least partly of a cylindrical shape. The parts are, typically, not completely of a cylindrical shape, the second part 3 does for instance comprise a mouthpiece, which has an oval shape. The first part 1 comprises an inner surface 2a with a circular cross section. This cross section 2a surrounds the outer surface 3a with a circular cross section of the second part 3. The two main parts 2, 3 are rotatable against each other in order for the patient to adjust the respiratory pressure. In a preferred embodiment the device 1 does not comprise any additional loose parts or elements. However, the two parts of figure 1 may comprise additional details such as means of attachment. The parts may be shaped in alternative designs. An advantage with the device is its non-complex design. As an example, the is no need for any separate part for adjustment of the respiratory pressure, instead the function for adjustment of the respiratory pressure is a part of the two at least partly cylindrical parts 2, 3.

Figure 1 further show that the first 2 and the second part 3 is dismountable from each other. A patient, pull the two parts from each other by hand, without using any tools. It is an advantage if the upper section of the first part 2 or the mouthpiece 4 of the second part 3 is shaped such that it is possible to place the device on a flat surface. In such a way the patient may use the flat surface as support.

Figure 1 further show that in the surface of the first part 2, as well as in the surface of the second part 3, there is a hole 6, 7 of a mainly triangular shape. These two mainly triangular shaped holes 6, 7, which are intended to be positioned opposite each other, form an opening 12 of variable size. When positioned against each other the two holes 6, 7 are in an angle such that the long side 6 of the first hole cuts the long side 7 of the second part in a near to 90 degrees angle.

From figure 3 it is seen that one of the holes 6, 7 is completely visible as the parts are assembled, and the two holes 6, 7 are positioned opposite each other. In the embodiment shown in figure 1-3 the hole is completely visible. In the embodiment the completely visible hole is positioned in the first part 2. The hole 6 and the resulting opening 12 is not only visible, but also possible to be felt by a finger tip of the patient. This enables a patient with defective vision to sense the size of the opening with the hole 6 as reference. The two holes 6, 7 may be shaped in alternative manner. Still the progressive effect is the same with an increase of the area 6 of the resulting opening, as the parts are rotated in even steps according to the scale 11.

The at least partly cylindrical shaped second part 3 comprises a longitudinal section with a circular cross section with a second outer surface 3b. This longitudinal section has a circular cross section with a diameter slightly larger than the cross section of the first outer surface 3a. The second outer surface 3b surrounds the first outer surface 3a. The surface connects to each other. The second part 3 comprises an elevation such that the lower part of the second part has a cross section diameter slightly larger than the cross section of the upper part. Due to the difference in diameter, the border between the lower section of the second part 3 and the upper section of the second part 3 is an edge. The edge prevents that the first part 2 may be pushed over the send part's outer surface 3b. When the two parts 2, 3 are assembled the first part 2 outer surface 2b and the second part 3 outer surface 3b are on the same level. The two parts 2, 3 are made such that the two outer surfaces 2b, 3b are suitable to be positioned such the bars of the scale 11 and the corresponding indication faces each other. Such a scale 11 is shown in figure 2. The scale 11 is intended to be used for adjusting the area of the opening 12 of variable size. The scale 11 comprises a number of indications 10 on the outer wall 2b of the first part 2, and at least one indication 9 on the second outer surface 3b of the second part 2. Figure 2 shows that the indications 10 may be shaped as bars. The indications are ordered in an increasing manner with even distances. The indications are elevated or immersed, which enables a patient to feel the scale with the finger tips.

The device may in addition to the indications of increasing length also comprise readable marks. Such marks may be numerical such as 1, 2, 3, 4 etc. Marks may also be based on characters A, B, C, D, E etc.

Figure 4 is a bar diagram where each bar shows an increase of the size of the opening 6 as the two parts are rotated, and the reference of the scale is moved from one indication to the next. The size of the opening 12 increases progressively when adjusting from the shortest indications of the scale to the longer indications of the scale 11. There is an increase of enlargement of size as the reference of the scale moves towards longer bars of the scale. Figure 4 shows that the increase of the area when rotating the indication from A to B is smaller than rotating from C to D. The patient is instructed to start therapy with a large opening corresponding to a relative small expiratory resistance. The opening is gradually decreased during a therapy. A decrease of the size of opening is typically reciprocally proportional to the increase of expiratory resistance.

The inner surface 2a of the first part may comprise an elevation 5a. Such an elevation 5a follows the full circle of the inner surface 2a. Such an elevation has a corresponding slit 5b in the full circle of the outer surface 3a of the second cylindrical part 3, wherein the two parts 2, 3 are held in place in the longitudinal direction of the device. As an alternative the slit may be arranged on the first part 2 and the elevation may be arranged on the second part 3. The first 2 and the second 3 part are dismountable from each other by pulling them in an opposite direction in a longitudinal direction. The elevation 5a and slit 5b are shaped such that the patient may pull the parts apart by hand.

A device according to the invention does typically not have moving parts or dismountable parts in the inner of the device where the exhaled air from the patient is intended to pass. Compared to previous known apparatuses the cleaning of the device is made in a more efficient manner. The device according to the invention is straightforward to dismount and to assemble after the cleaning or disinfection has been performed. The patient does not need any additional tools other than his hands to dismount and to assemble the device. Since the device typically does not have any additional parts, other than the first and second part. The patient handles cleaning of the device at home without any risk of loosing small parts while cleaning the device or assembles such small parts in an incorrect manner.

The device comprises two main parts, which reduces the manufacturing cost compared to previous known devices. The two at least partly cylindrical details may be manufactured by means of injection molding or casting. The material to be used is advantageously a polymer. During the manufacturing process the polymer is typically heated until it is plastic and then used to form the parts of the device.

## Claims

1. A device (1) for respiratory therapy, the device (1) comprises a first (2) and a second (3) part, wherein the second part (3) comprises a mouthpiece (4), each of said first part (2) and second part (3) at least partly of a cylindrical shape, the first part (2) comprises an inner surface (2a) with a circular cross section which surrounds a first outer surface (3a) with a circular cross section of the second part (3), the two parts (2, 3) are rotatable relative each other and the two parts (2, 3) are dismountable from each other, the surface (2a) of the first part (2) comprises a mainly triangular shaped hole (6), the first surface (3a) of the second part (3) comprises a mainly triangular shaped hole (7), the two holes (6, 7) are positioned against each other forming a resulting opening (12) with an adjustable area, where the enlargement of the opening (12) increases in a progressive manner as the two parts (2, 3) are turned in one direction, and the reduction of size of the opening (12) increases as the two parts (2, 3) are turned in the opposite direction, the mainly triangular shaped hole (6) of the first part and the resulting opening (12) are visible.

2. A device (1) according to claim 1 where the at least partly cylindrical shaped second part (3) in its longitudinal direction comprises a section with a second outer surface (3b) with a circular cross section greater than the cross section of the first outer surface (3a), the second outer surface (3b) connects to the first outer surface (3a) by an edge shaped connection, the device (1) comprises a scale (11) intended to facilitate adjustment of the area of the opening (12) of variable size, the scale (11) comprises a number of indications (10) on the outer surface (2b) of the first (2) at least partly cylindrically shaped part, alternatively on the outer surface (3b) of the second at least partly cylindrically shaped part (3), a reference indication (9) is arranged on the second outer surface (3b) of the second part (3), alternatively on the outer surface (2b) of the first part (2), the indications (10) are in the shape of bars and arranged in increasing lengths with equal distance from each other, and when the longest indication (E) of the scale (11) is positioned opposite the reference indication (9) the size of the area of the opening (12) is large, which corresponds to a small positive expiratory pressure.

3. A device according to claim 2 where the size of the opening (12) is configured to increase progressively as the parts (1, 2) are rotated such the indications (10) of the scale (11) goes from shorter to longer.

4. A device according to claim 3 where the inner surface (2a) of the first part (2) comprises an elevation (5a), alternatively a slit (5b), in the circumference of the inner surface (2a), which has a corresponding slit (5b), alternatively an elevation (5b) in the circumference of the outer surface (3a) of the second part (3), wherein the two parts (2, 3) are held together in a longitudinal direction during a therapy session.

5. A device according to claim 1 or 4 where the first (2) and second part (3) are dismountable from each other without the use of tools.

6. A device according to claim 1 where the second part (3) is molded in one piece.

7. A device according to claim 6 where the first part (2) is molded in one piece.

## Patentansprüche

1. Vorrichtung (1) zur Atmungstherapie, wobei die Vorrichtung (1) ein erstes Teil (2) und ein zweites Teil (3) umfasst, wobei das zweite Teil (3) ein Mundstück (4) umfasst, das erste Teil (2) und das zweite Teil (3) zumindest teilweise eine zylindrische Form aufweisen, das erste Teil (2) eine Innenfläche (2a) mit einem kreisförmigen Querschnitt, die eine erste Außenfläche (3a) des zweiten Teils (3) mit einem kreisförmigen Querschnitt umgibt, umfasst, die zwei Teile (2, 3) in Bezug zueinander drehbar sind und die zwei Teile (2, 3) voneinander abmontierbar sind, die Oberfläche (2a) des ersten Teils (2) ein im Wesentlichen dreieckig geformtes Loch (6) umfasst, die erste Oberfläche (3a) des zweiten Teils (3) ein im Wesentlichen dreieckig geformtes Loch (7) umfasst, die zwei Löcher (6, 7) aneinander angeordnet sind, um eine dadurch entstehende Öffnung (12) mit einem einstellbaren Bereich zu bilden, wobei die Öffnung (12) schrittweise vergrößert wird, wenn die zwei Teile (2, 3) in einer Richtung gedreht werden, und die Größe der Öffnung (12) verringert wird, wenn die zwei Teile (2, 3) in der entgegengesetzten Richtung gedreht werden, wobei das im Wesentlichen dreieckig geformte Loch (6) des ersten Teils und die dadurch entstehende Öffnung (12) sichtbar sind.

2. Vorrichtung (1) nach Anspruch 1, wobei das zumindest teilweise zylindrisch geformte zweite Teil (3) in seiner Längsrichtung einen Abschnitt mit einer zweiten Außenfläche (3b) mit einem kreisförmigen Querschnitt, der größer als der Querschnitt der ersten Außenfläche (3a) ist, umfasst, die zweite Außenfläche (3b) über eine kantenförmige Verbindung mit der ersten Außenfläche (3a) verbunden ist, die Vorrichtung (1) eine Skala (11) umfasst, die dazu dient, eine Einstellung des Bereichs der Öffnung (12) auf verschiedene Größen zu erleichtern, wobei die Skala (11) eine Anzahl von Anzeigen (10) auf der Außenfläche (2b) des ersten zumindest teilweise zylindrisch geformten Teils (2), alternativ auf der Außenfläche (3b) des zweiten zumindest teilweise zylindrisch geformten Teils (3), umfasst, eine Referenzanzeige (9) auf der zweiten Außenfläche (3b) des zweiten Teils (3), alternativ auf der Außenfläche (2b) des ersten Teils (2), angeordnet ist, die Anzeigen (10) die Form von Balken aufweisen und mit größer werdenden Längen in gleichem Abstand zueinander angeordnet sind, und wenn die längste Anzeige (E) der Skala (11) gegenüber der Referenzanzeige (9) angeordnet ist, der Bereich der Öffnung (12) groß ist, was einem kleinen positiven Ausatmungsdruck entspricht.

3. Vorrichtung nach Anspruch 2, wobei sich die Größe der Öffnung (12) schrittweise vergrößert, wenn die Teile (1, 2) gedreht werden, so dass sich die Anzeigen (10) der Skala (11) von kürzer zu länger verändern.

4. Vorrichtung nach Anspruch 3, wobei die Innenfläche (2a) des ersten Teils (2) eine Erhebung (5a), alternativ einen Schlitz (5b), im Umfang der Innenfläche (2a) umfasst, die bzw. der einen entsprechenden Schlitz (5b), alternativ eine Erhöhung (5b), im Umfang der Außenfläche (3a) des zweiten Teils (3) aufweist, wodurch die zwei Teile (2, 3) während einer Therapiesitzung in einer Längsrichtung zusammengehalten werden.

5. Vorrichtung nach Anspruch 1 oder 4, wobei das erste Teil (2) und das zweite Teil (3) ohne die Verwendung von Werkzeugen voneinander abmontierbar sind.

6. Vorrichtung nach Anspruch 1, wobei das zweite Teil (3) aus einem Stück geformt ist.

7. Vorrichtung nach Anspruch 6, wobei das erste Teil (2) aus einem Stück geformt ist.

## Revendications

1. Dispositif (1) pour thérapie respiratoire, le dispositif (1) comprend une première (2) et une seconde (3) parties, dans lequel la seconde partie (3) comprend un embout buccal (4), chacune desdites première partie (2) et seconde partie (3) étant au moins partiellement de forme cylindrique, la première partie (2) comprend une surface intérieure (2a) ayant une section transversale circulaire qui entoure une première surface extérieure (3a) ayant une section transversale circulaire de la seconde partie (3), les deux parties (2, 3) peuvent tourner l'une par rapport à l'autre et les deux parties (2, 3) peuvent être démontées l'une de l'autre, la surface (2a) de la première partie (2) comprend un trou principalement de forme triangulaire (6), la première surface (3a) de la seconde partie (3) comprend un trou principalement de forme triangulaire (7), les deux trous (6, 7) sont positionnés l'un contre l'autre formant une ouverture résultante (12) ayant une superficie réglable, où l'agrandissement de l'ouverture (12) augmente de manière progressive quand les deux parties (2, 3) sont tournées dans une direction, et la réduction de la taille de l'ouverture (12) augmente quand les deux parties (2, 3) sont tournées dans la direction opposée, le trou principalement de forme triangulaire (6) de la première partie et l'ouverture résultante (12) sont visibles.

2. Dispositif (1) selon la revendication 1 où la seconde partie de forme au moins partiellement cylindrique (3) dans sa direction longitudinale comprend une section avec une seconde surface extérieure (3b) ayant une section transversale circulaire supérieure à la section transversale de la première surface extérieure (3a), la seconde surface extérieure (3b) se connecte à la première surface extérieure (3a) par une connexion en forme de bord, le dispositif (1) comprend une échelle graduée (11) destinée à faciliter le réglage de la superficie de l'ouverture (12) de taille variable, l'échelle graduée (11) comprend un certain nombre d'indications (10) sur la surface extérieure (2b) de la première partie de forme au moins partiellement cylindrique (2), en variante sur la surface extérieure (3b) de la seconde partie de forme au moins partiellement cylindrique (3), une indication de référence (9) est agencée sur la seconde surface extérieure (3b) de la seconde partie (3), en variante sur la surface extérieure (2b) de la première partie (2), les indications (10) se présentent sous la forme de barres et sont agencées en longueurs croissantes à égale distance les unes des autres, et quand l'indication la plus longue (E) de l'échelle graduée (11) est positionnée à l'opposé de l'indication de référence (9), la taille de la superficie de l'ouverture (12) est grande, ce qui correspond à une faible pression expiratoire positive.

3. Dispositif selon la revendication 2 où la taille de l'ouverture (12) est configurée pour augmenter progressivement quand les parties (1, 2) sont tournées de telle sorte que les indications (10) de l'échelle graduée (11) aillent de la plus courte à la plus longue.

4. Dispositif selon la revendication 3 où la surface intérieure (2a) de la première partie (2) comprend une élévation (5a), en variante une fente (5b), dans la circonférence de la surface intérieure (2a), qui comporte une fente correspondante (5b), en variante une élévation (5b) dans la circonférence de la surface extérieure (3a) de la seconde partie (3), dans lequel les deux parties (2, 3) sont maintenues ensemble dans une direction longitudinale durant une session de thérapie.

5. Dispositif selon la revendication 1 ou 4 où les première (2) et seconde (3) parties peuvent être démontées l'une de l'autre sans l'utilisation d'outils.

6. Dispositif selon la revendication 1 où la seconde partie (3) est moulée d'un seul tenant.

7. Dispositif selon la revendication 6 où la première partie (2) est moulée d'un seul tenant.
